# EUROPEAN PATENT APPLICATION

(11) **EP 4 596 703 A1**
(43) Date of publication of application: **06.08.2025**
(21) Application number: 23872556.8
(22) Date of filing: 28.09.2023
(51) Int. Cl.: C12P 19/04, A23L 33/10, A61K 31/715, A61K 35/747, A61P 37/04, C08B 37/00, C12N 1/20, C12N 5/071

(54) **ACIDIC BACTERIAL EXOPOLYSACCHARIDE HAVING IMMUNOPOTENTIATING ACTIVITY**

(30) Priority: 29.09.2022 JP 2022155708
(71) Applicant: Meiji Holdings Co., Ltd., Tokyo 104-0031 (JP); INSTITUT PASTEUR, 75724 Paris Cedex 15 (FR); Centre National de la Recherche Scientifique, 75794 Paris Cedex 16 (FR); Institut National de la Santé et de la Recherche Médicale, 75654 Paris Cedex 13 (FR)
(72) Inventor: ASAMI, Yukio, Hachioji-shi, Tokyo 192-0919 (JP); MAKINO, Seiya, Hachioji-shi, Tokyo 192-0919 (JP); EBERL, Gerard, 75724 Paris Cedex 15 (FR); GOMPERTS BONECA, Ivo, 75724 Paris Cedex 15 (FR); RIFFLET, Aline, 75724 Paris Cedex 15 (FR)
(74) Representative: TBK
(86) International application number: PCT/JP2023/035511
(87) International publication number: WO 2024/071336

(57) **Abstract**

The present invention relates to an exopolysaccharide having a repeating structure comprising a string of repeating units of the following formula (I), wherein in the formula (I), n denotes an integer of 0 or 1, independently for each repeating unit; and its intended use for immunostimulation:

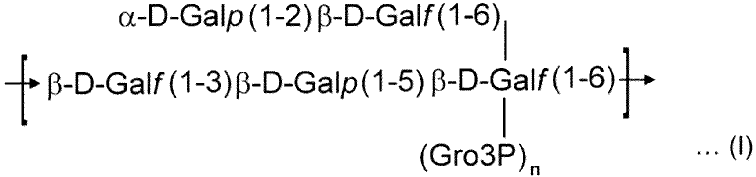

## Description

### Technical Field

The present invention relates to an acidic exopolysaccharide having immunostimulatory activity.

### Background Art

Various microorganisms, including lactic acid bacteria, are known to produce and extracellularly secrete exopolysaccharides (EPS). EPSs are also contained in fermented milk, including yogurt, which is produced by fermenting milk using lactic acid bacteria *Lactobacillus delbrueckii* ssp. (or subsp.) *bulgaricus* and *Streptococcus thermophilus.* In recent years, EPSs have received attention for their various physiologically active functions, such as their immunostimulatory function.

The EPS produced by *Lactobacillus delbrueckii* ssp. *bulgaricus* strain OLL1073R-1 is known to have a repeating structure comprising a string of many repeating units, each of which is composed of a main chain comprising two glucose residues and two galactose residues and a side chain comprising one galactose residue, and to include acidic polysaccharides having the repeating structure with phosphate groups linked thereto and neutral polysaccharides having the repeating structure with no phosphate groups linked thereto (Non Patent Literatures 1 and 2). Furthermore, such acidic EPS produced by the strain OLL1073R-1 is known to have an immunostimulatory action (Non Patent Literatures 1 and 2).

On the other hand, there is a further demand for obtaining novel EPSs with immunostimulatory action.

### Citation List

### Non Patent Literature

Non Patent Literature 1: Marie-Rose Van Calsteren, et al., Carbohydrate Research, 413 (2015) 115-122
Non Patent Literature 2: Seiya Makino, Milk Science, Vol. 58, No. 2 (2009)

### Summary of Invention

### Technical Problem

An object of the present invention is to further provide a polysaccharide having immunostimulatory activity.

### Solution to Problem

As a result of extensive studies to solve the above problem, the present inventors have succeeded in obtaining an exopolysaccharide (EPS) having immunostimulatory activity, which has a novel repeating structure comprising a string (repetition) of repeating units in which the sugar residues are only galactose residues, from the culture of strain OLL1073R-1, and thereby completed the present invention.

That is, the present invention includes the following aspects.
[1] An acidic exopolysaccharide having a repeating structure comprising a string of repeating units of the following formula (I): 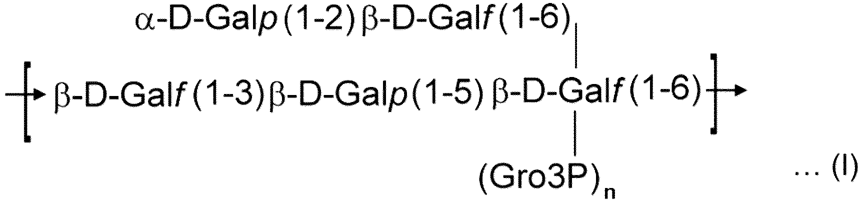 wherein in the formula (I), n denotes an integer of 0 or 1, independently for each repeating unit.
[2] The acidic exopolysaccharide according to the above [1], wherein the exopolysaccharide is isolated.
[3] The acidic exopolysaccharide according to the above [1] or [2], wherein the exopolysaccharide is isolated from a lactic acid bacterial culture comprising *Lactobacillus delbrueckii* subsp. *bulgaricus.*
[4] The acidic exopolysaccharide according to any of the above [1] to [3], wherein the exopolysaccharide is derived from *Lactobacillus delbrueckii* subsp. *bulgaricus* strain OLL1073R-1 (accession number FERM BP-10741).
[5] A composition for immunostimulation, comprising the acidic exopolysaccharide according to any of the above [1] to [4] as an active ingredient.
[6] A food or a medicament for immunostimulation, comprising the acidic exopolysaccharide according to any of the above [1] to [4] as an active ingredient.
[7] A method for immunostimulation, comprising feeding or administering to a subject the acidic exopolysaccharide according to any of the above [1] to [4], the composition for immunostimulation according to the above [5], or the food or medicament for immunostimulation according to the above [6].
[8] Use of the acidic exopolysaccharide according to any of the above [1] to [4], in the manufacture of a composition, food or medicament for immunostimulation.

The present specification includes the disclosures in the Japanese Patent Application No. 2022-155708, from which he present application claims priority.

### Advantageous Effects of Invention

The present invention can provide a novel EPS having immunostimulatory activity.

### Brief Description of Drawings

[Figure 1] Figure 1 is a diagram showing the spectrum obtained by the [¹H-¹³C]-HSQC method for the acidic EPS obtained in Example 1.
[Figure 2] Figure 2 is a diagram showing the spectrum obtained by the [¹H-¹H]-NOESY method for the acidic EPS obtained in Example 1.
[Figure 3] Figure 3 is a diagram showing the repeating structure of the acidic EPS obtained in Example 1.
[Figure 4] Figure 4 is a graph showing the results of inducing IFN-y production in splenocytes by stimulation with the acidic EPS obtained in Example 1. "*" in the diagram indicates that there is a statistically significant difference.

### Description of Embodiments

The following describes in detail the present invention.

The present invention relates to an acidic exopolysaccharide (EPS) having an immunostimulatory action, and to the intended use of the exopolysaccharide for immunostimulation.

The acidic EPS of the present invention is a polysaccharide that has a repeating structure (sugar chain) comprising a string of repeating units, each of which is composed of a main chain comprising three galactose residues and a side chain comprising two galactose residues, and that has at least one glycerol phosphate group linked to the repeating structure. In the present invention, the term "a string of repeating units" means that the repeating units are arranged in tandem.

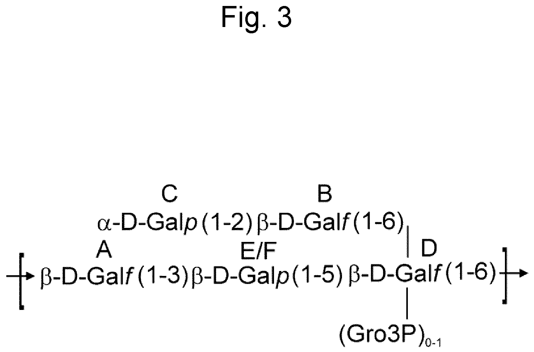

More specifically, the acidic EPS of the present invention is an acidic exopolysaccharide having a repeating structure comprising a string of repeating units of the following formula (I) (see also Figure 3).

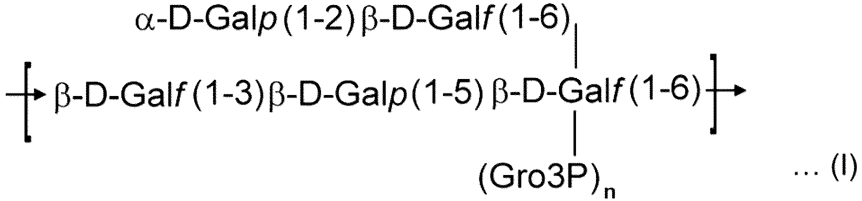

In formula (I), n denotes an integer of 0 or 1, independently for each repeating unit. That is, the individual repeating units of formula (I) that constitute the acidic EPS of the present invention have one or no glycerol 3-phosphate group, but the acidic EPS as a whole has at least one glycerol 3-phosphate group linked thereto.

The acidic EPS of the present invention may be an acidic exopolysaccharide having a repeating structure of the following formula (II), which comprises a string of repeating units of the formula (I).

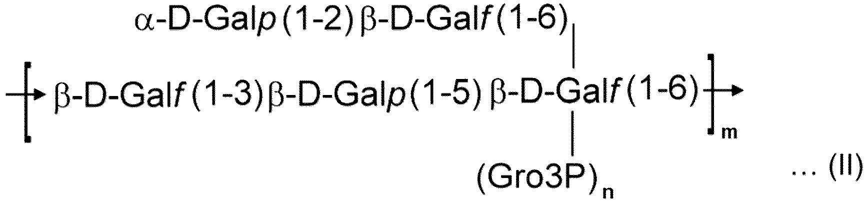

In formula (II), n denotes an integer of 0 or 1, independently for each repeating unit. In formula (II), m denotes an integer of 1 to 300, and preferably an integer of 1 to 200.

In formulae (I) and (II), α-D-Galp denotes an α-D-galactose residue in pyranose form, β-D-Galp denotes a β-D-galactose residue in pyranose form, β-D-Galf denotes a β-D-galactose residue in furanose form, and Gro3P denotes a glycerol 3-phosphate group. (1-2), (1-3), (1-5), and (1-6) in formulae (I) and (II) denote 1-2 linkage (i.e., 1-position carbon - 2-position carbon linkage), 1-3 linkage, 1-5 linkage, and 1-6 linkage between residues, respectively.

The acidic EPS of the present invention preferably has an average of about 1 glycerol 3-phosphate group linked per repeating unit (e.g., 1 (n = 1) or 0 per each repeating unit, and a weighted average of about 1 per repeating unit for the whole acidic EPS), in the repeating structure (e.g., formula (II)) comprising a string of repeating units of formula (I), but is not limited thereto.

The acidic EPS of the present invention preferably has the above-mentioned repeating structure as its basic backbone. In the present invention, "having the repeating structure as the basic backbone" means that the entire length or almost the entire length of the acidic EPS (80% or more, preferably 90% or more, more preferably 95% or 98% or more of the total number of sugar residues constituting the main chain of the acidic EPS) is composed of a repeating structure(s) comprising a string of repeating units of formula (I). In one embodiment, the acidic EPS of the present invention may solely consist of the above-mentioned repeating structure.

The acidic EPS of the present invention may be derived from microorganisms (typically, bacteria) including lactic acid bacteria. The acidic EPS of the present invention may be a product produced and extracellularly secreted by microorganisms (typically, bacteria) including lactic acid bacteria.

In one embodiment, the acidic EPS of the present invention may be obtained from a culture of microorganisms, or obtained from a lactic acid bacterial culture, and for example, may be collected or purified from a lactic acid bacterial culture. In one embodiment, the acidic EPS of the present invention may be isolated, for example, from a lactic acid bacterial culture. The term "isolated" with respect to the acidic EPS of the present invention means that the purity of the acidic EPS of the present invention is 80 mass% or more, preferably 90 mass% or more, and more preferably 98 mass% or more.

Examples of lactic acid bacteria include, but are not limited to, bacteria of the genus *Lactobacillus, Streptococcus, Lactococcus, Leuconostoc, Enterococcus,* and *Pediococcus.* Examples of lactic acid bacteria include, but are not limited to, *Lactobacillus delbrueckii subsp. bulgaricus, Lactobacillus casei, Lactobacillus gasseri, Lactobacillus fermentum, Lactobacillus helveticus, Lactobacillus acidophilus, Lactobacillus plantarum, Lactobacillus rhamnosus, Lactobacillus pentosus, Lactobacillus paracasei, Lactobacillus kefiranofaciens, Lactobacillus sake, Streptococcus thermophilus, Lactococcus lactis* ssp. *lactis, Lactococcus lactis* ssp. *cremoris, Leuconostoc mesenteroides, Enterococcus faecalis,* and *Pediococcus pentosaceus.*

More preferred examples of lactic acid bacteria used to produce the acidic EPS of the present invention include *Lactobacillus delbrueckii* subsp. *bulgaricus,* and particularly preferred examples of lactic acid bacteria include *Lactobacillus delbrueckii* subsp. *bulgaricus* strain OLL1073R-1.

*Lactobacillus delbrueckii* subsp. *bulgaricus* strain OLL1073R-1 has been deposited internationally under the Budapest Treaty at the International Patent Organism Depositary, National Institute of Technology and Evaluation (NITE-IPOD) (the former name: the International Patent Organism Depositary, the National Institute of Advanced Industrial Science and Technology) (#120, 2-5-8, Kazusakamatari, Kisarazu-shi, Chiba, 292-0818, Japan) as of February 22, 1999 (date of the original deposit) under accession number FERM BP-10741. This deposited strain was transferred from the domestic deposit (original deposit) to international deposit under the Budapest Treaty, on November 29, 2006. The current depositor of *Lactobacillus delbrueckii* subsp. *bulgaricus* strain OLL1073R-1 is Meiji Co., Ltd. Meiji Co., Ltd. has authorised Meiji Holdings Co., Ltd. to refer to strain OLL1073R-1 in the present application.

In one embodiment, the acidic EPS of the present invention can be obtained from a lactic acid bacterial culture containing one or more species of the above-mentioned lactic acid bacteria. The lactic acid bacterial culture may be fermented milk such as yogurt produced using lactic acid bacteria, or obtained by culturing lactic acid bacteria in a culture medium (such as a milk-containing medium), but is not limited thereto. The "milk" that may be used for the preparation of the lactic acid bacterial culture in the present invention is preferably the milk of mammals (e.g., cow, goat, and sheep).

In one embodiment, the lactic acid bacterial culture may comprise bacteria of the genus *Lactobacillus* as lactic acid bacteria, for example, *Lactobacillus delbrueckii subsp. bulgaricus.*

In one embodiment, the lactic acid bacterial culture may comprise a combination of bacteria of the genus *Lactobacillus* and *Streptococcus* as lactic acid bacteria, and in a preferred embodiment, the lactic acid bacterial culture may comprise *Lactobacillus delbrueckii* subsp. *bulgaricus* and *Streptococcus thermophilus.*

In one embodiment, the acidic EPS of the present invention may be derived from lactic acid bacteria, for example, derived from the above-mentioned lactic acid bacteria such as bacteria of the genus *Lactobacillus* and/or *Streptococcus,* and is preferably derived from *Lactobacillus delbrueckii* subsp. *bulgaricus,* and more preferably from *Lactobacillus delbrueckii* subsp. *bulgaricus* strain OLL1073R-1.

The culture conditions used to produce the acidic EPS of the present invention by lactic acid bacteria may be, for example, the same culture conditions as in the Examples described below, but are not limited thereto. Such culture conditions may include, for example, culturing at 35 to 39°C (typically, 37°C) for 12 to 24 hours (e.g., 18 hours) in a milk-containing medium, such as skim milk medium.

A known technique for isolating and purifying polysaccharides can be used to obtain the acidic EPS of the present invention from the lactic acid bacterial culture. For example, the EPSs in lactic acid bacterial cultures can be separated from proteins and lactic acid bacteria by trichloroacetic acid treatment for collection and then after dialysis, the acidic EPS fraction can be separated using a fractionation technique such as anion exchange chromatography. Furthermore, the acidic EPS of the present invention can be obtained by removing the proteins remaining in the separated acidic EPS fraction using protein hydrolysis enzymes and the like, followed by further purification. However, the method for isolating and purifying the acidic EPS of the present invention is not limited to the method.

Alternatively, the acidic EPS of the present invention may be chemically synthesized using glycochemical synthesis. The chemically synthesized acidic EPS of the present invention may also be included in the scope of the "isolated" acidic EPS in the present invention.

The acidic EPS of the present invention has an immunostimulatory activity (immunostimulatory action). The acidic EPS of the present invention having immunostimulatory activity can preferably promote the production of interferon (IFN)-y. IFN-y is mainly produced by immune cells such as T cells and natural killer (NK) cells, and is known to contribute to antiviral, immunomodulatory, and antitumor activities, and to function as a potent activator of macrophages.

The immunostimulatory activity of the acidic EPS of the present invention can be evaluated using IFN-y production levels as an indicator. The immunostimulatory activity of the acidic EPS of the present invention can be evaluated, for example, using the same test method as in Example 3 described later. Specifically, mouse splenocytes are first suspended in a medium for mammalian cell culture (e.g., RPMI 1640 medium) containing fetal bovine serum (e.g., 10% (vol/vol) fetal bovine serum) and then seeded onto plates or the like at an appropriate cell density (e.g., 5 × 10⁵ cells/well). The acidic EPS is added to the seeded cells at a given concentration (e.g., a final concentration of 25 to 200 µg/mL), and then the cells are cultured at 37°C and 5% CO₂ for a certain period of time (e.g., 72 hours). The supernatant is then collected from the medium, and the level of IFN-γ in the collected supernatant is quantified by ELISA (enzyme-linked immunosorbent assay) using an anti-IFN-y antibody. As a control, mouse splenocytes are cultured in the same manner but without adding the acidic EPS, and the level of IFN-y in the supernatant is quantified, and then the level of IFN-y is compared with the level of IFN-y quantified as described above in the culture system with the acidic EPS. If the level of IFN-y in the supernatant statistically significantly increases when the acidic EPS is added compared to the control in which no acidic EPS is added, or if the level of IFN-γ in the supernatant increases in a concentration-dependent manner with increasing amounts of the acidic EPS added, the acidic EPS of the present invention can be determined as having immunostimulatory activity. In addition, the intensity (level) of the immunostimulatory activity of the acidic EPS of the present invention can be evaluated based on the amount of increase in the level of IFN-y in the supernatant when the acidic EPS is added compared to the control in which no acidic EPS is added.

In addition, the present invention also provides a composition comprising the acidic EPS of the present invention, and a food or medicament (pharmaceutical) comprising the acidic EPS of the present invention. The composition, food or medicament comprising the acidic EPS of the present invention preferably has an immunostimulatory activity, and is preferably for immunostimulation. In a preferred embodiment, the acidic EPS of the present invention can be comprised as an active ingredient (active ingredient for providing immunostimulatory activity) in a composition for immunostimulation and a food or medicament for immunostimulation. The acidic EPS of the present invention may be comprised as the sole active ingredient for immunostimulation, and/or as the sole EPS in a composition for immunostimulation and a food or medicament for immunostimulation. In the present invention, the phrase "comprising as an active ingredient" the acidic EPS of the present invention means that the acidic EPS of the present invention is comprised in a composition, food or medicament in an amount (effective amount) and form sufficient to exhibit immunostimulatory activity when the composition, food or medicament comprising the acidic EPS of the present invention is applied to a subject. The composition, food or medicament comprising the acidic EPS of the present invention may comprise, for example, the acidic EPS of the present invention in an amount ranging from 10 µg to 50 mg/kg body weight per application (ingestion or administration), but not limited thereto.

In one embodiment, the composition, food or medicament comprising the acidic EPS of the present invention can be free of neutral EPS (e.g., neutral EPS derived from lactic acid bacteria such as *Lactobacillus delbrueckii* subsp. *bulgaricus,* including strain OLL1073R-1). In one embodiment, the composition, food or medicament comprising the acidic EPS of the present invention can be free of an acidic EPS (e.g., one derived from lactic acid bacteria such as *Lactobacillus delbrueckii* subsp. *bulgaricus,* including strain OLL1073R-1) having a repeating structure comprising a string of repeating units, each of which is composed of a main chain comprising two glucose residues and two galactose residues and a side chain comprising one galactose residue, and having a phosphate group linked to the repeating structure.

In addition to the acidic EPS of the present invention, the composition, food or medicament of the present invention may comprise an additive acceptable for food processing (food additive) or a pharmaceutically acceptable additive (pharmaceutical additive). Examples of such additives include carriers, excipients, binders, lubricants, disintegrants, wetting agents, stabilizers, buffers, flavoring agents, preservatives, and coloring agents, but are not limited thereto. In one embodiment, the composition comprising the acidic EPS of the present invention (e.g., composition for immunostimulation) may be a food composition or a pharmaceutical composition, but is not limited thereto. The composition, food or medicament of the present invention may further comprise other pharmacological and/or functional ingredients.

**In** the present invention, the "food" may be a beverage or another food, but is preferably a processed food. Examples of beverages include, but are not limited to, beverages such as lactic acid bacteria beverages, milk beverages (coffee milk, fruit milk, etc.), tea beverages (green tea, black tea, oolong tea, etc.), fruit and vegetable beverages (beverages containing fruit juices such as orange, apple and grape, and/or vegetable juices such as tomato and carrot), alcoholic beverages (beer, sparkling liquor, wine, etc.), carbonated beverages, soft drinks, and water-based beverages. Examples of foods other than beverages include, but are not limited to, processed foods such as fermented milk such as yogurt, confectioneries, jams, prepared foods, instant foods, and seasonings. Existing reference books related to food production can be referred to for the methods for producing various foods and the like.

The food of the present invention may be a functional food or otherwise. In the present invention, a "functional food" means a food that has a property providing a certain function to a living body, and includes any so-called health foods, such as foods with health claims, including foods for specified health uses (including qualified foods for specified health uses) and foods with nutrient function claims in Japan, foods with functional claims, foods for special dietary uses, dietary supplements, health supplements, supplements (e.g., supplements in various dosage forms such as tablets, coated tablets, sugar-coated tablets, capsules, and liquids), and beauty foods (e.g., diet foods). In addition, the functional food of the present invention includes health foods to which health claims based on the Codex (FAO/WHO Joint Alimentarius Commission) food standards apply.

The functional food of the present invention may be a food for special dietary uses such as a food for the sick, milk powder for expectant, nursing or lactating mothers, formulated milk powder for infants, a food for the elderly, or a food for nursing care.

The functional food of the present invention may be a solid preparation such as a tablet, granules, a powder, a pill, or a capsule, a liquid preparation such as a liquid, a suspension, or a syrup, a gel, a paste, or the like, or it may be in a normal food form (e.g., beverage, yogurt, and confectionery).

The food of the present invention may contain any food ingredients in addition to the acidic EPS of the present invention, without limitation. The food of the present invention may contain water, proteins, carbohydrates, lipids, vitamins, minerals, organic acids, organic bases, fruit juices, flavors, and the like. Examples of proteins include animal and plant proteins such as whole milk powder, skim milk powder, partly skimmed milk powder, casein, β-lactoglobulin, α-lactalbumin, lactoferrin, soy protein, chicken egg protein, and meat protein, and hydrolysates thereof; and various milk-derived ingredients such as butter, whey minerals, cream, whey, non-protein nitrogen, sialic acid, phospholipids, and lactose. Examples of carbohydrates include common sugars, processed starches (dextrin, soluble starch, British starch, oxidized starch, starch esters, starch ethers, etc.), and dietary fiber. Examples of lipids include animal oils and fats such as lard, fish oil, and the fractionated, hydrogenated, and interesterified oils thereof; and vegetable oils and fats such as palm oil, safflower oil, corn oil, rapeseed oil, coconut oil, and the fractionated, hydrogenated, and interesterified oils thereof. Examples of vitamins include vitamin A, carotenes, vitamin B, vitamin C, vitamin D, vitamin E, vitamin K, vitamin P, vitamin Q, niacin, nicotinic acid, pantothenic acid, biotin, inositol, choline, and folic acid, and examples of minerals include calcium, potassium, magnesium, sodium, copper, iron, manganese, zinc, selenium, and whey minerals. Examples of organic acids include malic acid, citric acid, lactic acid, and tartaric acid. These components may be used alone or in combination of two or more, and synthetic products and/or food materials containing large amounts of these components may be used as raw materials. The food of the present invention can be produced by a conventional method, for example, by adding the acidic EPS of the present invention or a raw material containing the acidic EPS of the present invention to other food ingredients in any step of the food production.

The medicament of the present invention may be in any dosage form, such as a solid preparation such as a tablet, granules, a powder, a pill, or a capsule, a gel, or a liquid preparation such as a liquid, a suspension, or a syrup. The medicament of the present invention is preferably a medicament for oral administration, but is not limited thereto.

The composition, food or medicament of the present invention is capable of providing an immunostimulatory effect based on the immunostimulatory activity (immunostimulatory action) of the acidic EPS of the present invention. For example, the composition, food or medicament of the present invention can promote the production of interferon (IFN)-y. The composition, food or medicament of the present invention can promote (stimulate) an immune response in the subject who has ingested or administered it.

The present invention also provides a method for providing an immunostimulatory effect (i.e., a method for immunostimulation) in a subject, which comprises feeding or administering to the subject the acidic EPS of the present invention, or the composition, food or medicament of the present invention comprising the same.

The subject which is fed or administered the acidic EPS of present invention, or the composition, food or medicament of the present invention comprising the same is preferably a mammal including primates such as humans, livestock, pets, and laboratory (test) animals, and more preferably a human. The subject may be, but is not limited to, a subject in need of immunostimulation or a subject for whom immunostimulation is desired, and examples thereof include expectant or nursing mothers, infants, the elderly, the sick, the convalescent, the infirm, subjects with weakened immune systems, athletes, and subjects who need to be prevented from contracting diseases such as infectious diseases (e.g., healthcare professionals, nursing care workers, and students preparing for an exam).

The feeding or administration to a subject of the acidic EPS of the present invention, or the composition, food or medicament of the present invention comprising the same is preferably oral, but may be done via a tube such as a nasal tube, an oro-esophageal tube, a gastrostomy tube, or an enterostomy tube. However, the route of administration to the subject in the present invention is not necessarily limited thereto.

The feeding amount (intake) or administration amount (dosage) of the acidic EPS of the present invention, or the composition, food or medicament of the present invention comprising the same, should be an amount effective for immunostimulation, and can be varied widely at the discretion of the person skilled in the art, taking into consideration, e.g., the subject's species, age, weight, and the route and frequency of administration. In one embodiment, the intake or dosage may be an amount corresponding to 10 µg to 50 mg of the acidic EPS of the present invention per kg body weight per application (feeding or administration) based on the subject's body weight, but is not limited thereto.

The acidic EPS of the present invention, or the composition, food or medicament of the present invention comprising the same may be fed or administered in a single (one) dose only or a plurality of doses. The acidic EPS of the present invention, or the composition, food or medicament of the present invention comprising the same may be fed or administered to the subject continuously, or daily, or at a frequency of, for example, one, two, three, four, five, or six days per week. If fed or administered continuously, it is preferably fed or administered to the subject for at least 3 days, preferably 1 week or more, more preferably 2 weeks or more, and even more preferably 4 weeks or more, such as 3 months or more.

The present invention also relates to the acidic EPS of the present invention for use in immunostimulation (promotion of immune response). Moreover, the present invention also relates to use of the acidic EPS of the present invention for immunostimulation (promotion of immune response). Furthermore, the present invention also relates to use of the acidic EPS of the present invention in the manufacture of a composition, food or medicament for immunostimulation (promotion of immune response).

### Examples

Hereinafter, the present invention will be described more specifically using Examples. However, the technical scope of the present invention is not limited to these Examples.

### [Example 1] Production and purification of acidic EPS

The lactic acid bacteria *Lactobacillus delbrueckii* ssp. *bulgaricus* strain OLL1073R-1 (accession number FERM BP-10741) was cultured in a 10% (wt/vol) skim milk medium at 37°C for 18 hours. Trichloroacetic acid was added to the resulting culture at a final concentration of 10 mass%, and the generated denatured proteins and lactic acid bacteria were precipitated, then centrifuged (12,000 × g, 20 min). The resulting supernatant was collected and a 3-fold volume of cold ethanol was added. The mixture was allowed to stand overnight at 4°C. The resulting precipitate was collected by centrifugation (12,000 × g, 20 min) and dialyzed with distilled water using a dialysis membrane (MWCO [molecular weight cut off] 6,000 to 8,000). This yielded crude EPS (exopolysaccharide).

The acidic EPS was fractionated from the crude EPS obtained. For fractionation, the anion exchange carrier DEAE-Sepharose^{(R)} (Diethylaminoethyl-Sepharose^{(R)}) Fast Flow was used, and a gradient extraction method with 0 to 0.5 M NaCl in a 0.02 M Tris-HCl buffer (pH 8.6) was used. The resulting acidic EPS was dissolved in 0.05M Tris-HCl buffer (pH 8.0) containing 1 mM MgCl₂ and treated with 2mg/mL DNase and 2mg/mL RNase at 37°C for 6 hours. Then, it was treated at 37°C for 16 hours by adding a proteinase K solution at a final concentration of 0.1 mg/mL to degrade proteins, and further heated at 90°C for 10 minutes to inactivate the enzyme. To the resulting reaction solution, in the same manner as described above, a 3-fold volume of cold ethanol was added and allowed to stand overnight at 4°C, and the resulting precipitate was collected by centrifugation (12,000 × g, 20 min), and then dialyzed with distilled water using the above-mentioned dialysis membrane, repeatedly, thereby purifying the acidic EPS to prepare an acidic EPS solution.

### [Example 2] Structure analysis of acidic EPS

A structural analysis of the purified acidic EPS was performed by two-dimensional NMR. The two-dimensional NMR used for the analysis is a nuclear magnetic resonance (NMR) spectroscopy method that allows for structural analysis of complex compounds by identifying correlations between signals, specifically, [¹H,¹H]-COSY (¹H,¹H-correlation spectroscopy), TOCSY (total correlation spectroscopy), [¹H-¹³C]-high resolution two-dimensional NMR ([¹H-¹³C]-heteronuclear multiple quantum correlation: [¹H-¹³C]-HSQC), and [¹H,¹H]-NOESY ([¹H,¹H]-nuclear overhauser enhancement spectroscopy).

The structure of the acidic EPS was first estimated using [¹H,¹H]-COSY, because the signal of each proton is affected by the two substituents bound to the same carbon atom (in geminal positions) or the two substituents bound to adjacent carbon atoms (in vicinal positions), and thus are assigned to the ¹H chemical shifts of almost all anomeric sugars. Furthermore, since oligosaccharides and polysaccharides have complex overlapping signals, the data obtained by [¹H,¹H]-COSY was supplemented with data using TOCSY, which can extract from the signals the correlation peaks of all protons belonging to a specific spin system. On the other hand, [¹H-¹³C]-HSQC, which can detect correlations between two different types of nuclei, such as hydrogen and carbon atoms, was used to analyze which hydrogen atom was directly bound to which carbon atom. Furthermore, [¹H,¹H]-NOESY, which observes signals between spatially proximal protons, was used to determine which signals were derived from protons that are proximal to each other.

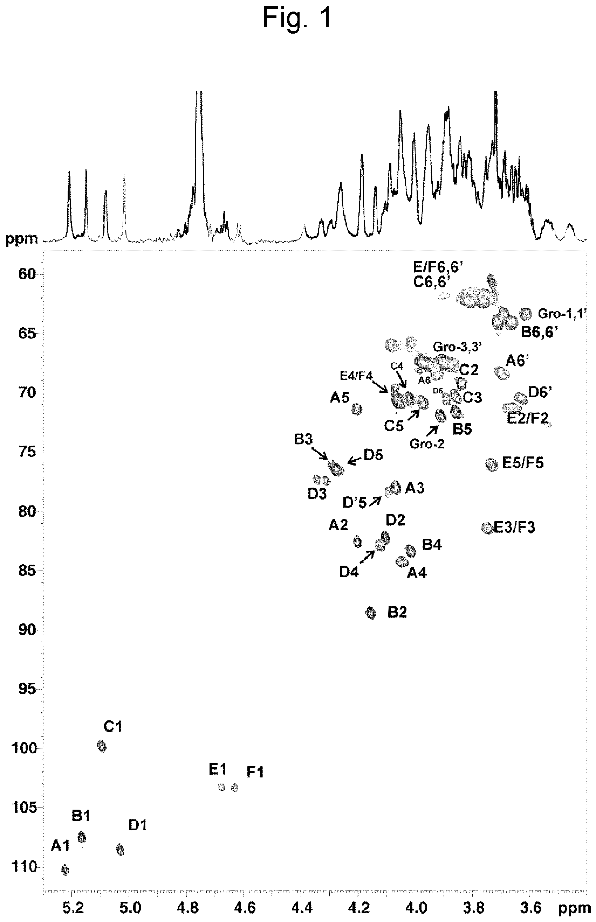

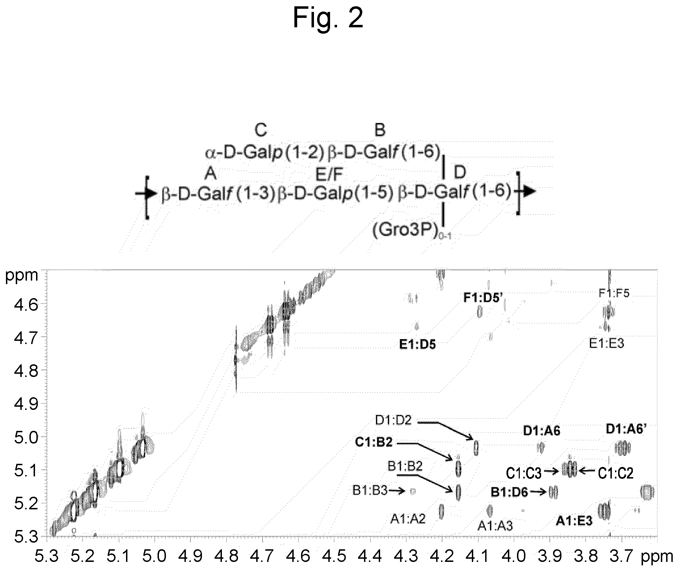

The spectrum obtained by [¹H-¹³C]-HSQC is shown in Figure 1, and the spectrum obtained by [¹H-¹H]-NOESY is shown in Figure 2.

The results of the two-dimensional NMR structural analysis showed that the obtained acidic EPS has mainly a repeating structure comprising a string of repeating units, each of which comprises galactose residues (three galactose residues in furanose form and two galactose residues in pyranose form). Furthermore, it was also shown that in many of the repeating units in this repeating structure, one glycerol phosphate group per repeating unit is linked, which forms an anchor to the bacterial cell wall.

Furthermore, for glycosyl linkage analysis, the obtained acidic EPS was permethylated, depolymerized, and acetylated, and the resulting methylated alditol acetates were analyzed by gas chromatography-mass spectrometry (GC-MS) to determine the repeating structure of the acidic EPS as shown in Figure 3. In Figure 3, A, B, C, D, and E/F are symbols assigned for convenience to refer to the respective sugar residues indicated in the spectra of Figures 1 and 2. In the structure shown in Figure 3, Galp denotes a galactose residue in pyranose form, Galf denotes a galactose residue in furanose form, and Gro3p denotes a glycerol 3-phosphate group.

### [Example 3] Evaluation of immunostimulatory activity of acidic EPS

Mouse splenocytes were suspended in RPMI 1640 medium containing 10% (vol/vol) fetal bovine serum and then seeded on a 96-well plate at a density of 5 × 10⁵ cells/well. A solution of the acidic EPS which had been purified in Example 1 and whose structure had been determined in Example 2, was added to the seeded cells at a final concentration of 25 µg/mL, 50 µg/mL, 100 µg/mL, or 200 µg/mL of EPS, and the cells were cultured at 37°C and 5% CO₂ for 72 hours (acidic EPS stimulated group). Further, as a control, mouse splenocytes were cultured in the same manner but without adding the acidic EPS (unstimulated group). After culture, the supernatant was collected and the level of interferon y (IFN-y) produced in the medium was determined by ELISA.

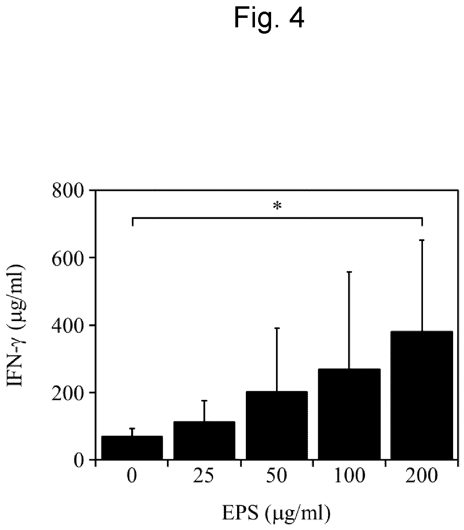

As a result, IFN-y production in splenocytes was induced in an amount depending on the concentration of acidic EPS added (Figure 4). This showed that the obtained acidic EPS is capable of activating immune cells.

All publications, patents and patent applications cited in the present specification are hereby incorporated by reference in their entirety.

## Claims

1. An acidic exopolysaccharide having a repeating structure comprising a string of repeating units of the following formula (I): 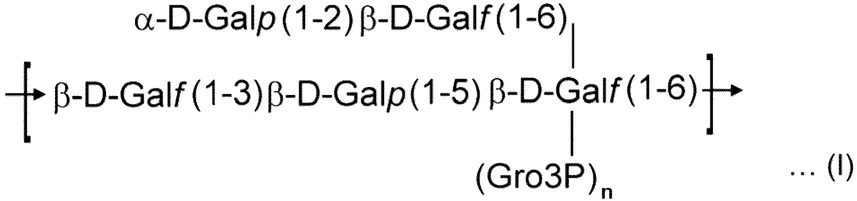 wherein in the formula (I), n denotes an integer of 0 or 1, independently for each repeating unit.

2. The acidic exopolysaccharide according to claim 1, wherein the exopolysaccharide is isolated.

3. The acidic exopolysaccharide according to claim 1 or 2, wherein the exopolysaccharide is isolated from a lactic acid bacterial culture comprising *Lactobacillus delbrueckii* subsp. *bulgaricus.*

4. The acidic exopolysaccharide according to claim 1, wherein the exopolysaccharide is derived from *Lactobacillus delbrueckii* subsp. *bulgaricus* strain OLL1073R-1 (accession number FERM BP-10741).

5. A composition for immunostimulation, comprising the acidic exopolysaccharide according to claim 1 as an active ingredient.

6. A food or a medicament for immunostimulation, comprising the acidic exopolysaccharide according to claim 1 as an active ingredient.

7. A method for immunostimulation, comprising feeding or administering to a subject the acidic exopolysaccharide according to claim 1, the composition for immunostimulation according to claim 5, or the food or medicament for immunostimulation according to claim 6.

8. Use of the acidic exopolysaccharide according to claim 1, in the manufacture of a composition, food or medicament for immunostimulation.
